Europäisches Patentamt

European Patent Office

Office européen des brevets

(19)

(11) Veröffentlichungsnummer: **0 022 161**
**B1**

(12) # EUROPÄISCHE PATENTSCHRIFT

(45) Veröffentlichungstag der Patentschrift:
**10.11.82**

(21) Anmeldenummer: **80102814.3**

(22) Anmeldetag: **21.05.80**

(51) Int. Cl.³: **C 07 D 201/16**

(54) **Verfahren zur Reinigung von Caprolactam.**

(30) Priorität: **29.06.79 DE 2926279**

(43) Veröffentlichungstag der Anmeldung:
**14.01.81 Patentblatt 81/2**

(45) Bekanntmachung des Hinweises auf die Patenterteilung:
**10.11.82 Patentblatt 82/45**

(84) Benannte Vertragsstaaten:
**BE CH DE FR GB IT LI NL**

(56) Entgegenhaltungen:
**DD-A-79 494**
**DE-A-1 770 745**
**DE-A-2 163 258**
**DE-A-2 801 256**
**DE-C-920 073**
**DE-C-1 194 863**
**FR-A-1 337 717**
**US-A-2 758 991**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Fuchs, Hugo, Dr., Egellstrasse 28,
D-6700 Ludwigshafen (DE)**
Erfinder: **Grosskinsky, Otto-Alfred, Dr.,
Semmelweisstrasse 8, D-6700 Ludwigshafen (DE)**
Erfinder: **Frommer, Elmar, DR., Lisztstrasse 117,
D-6700 Ludwigshafen (DE)**
Erfinder: **Kartte, Klaus, Dr., Stettiner Strasse 1,
D-6711 Beindersheim (DE)**

Verfahren zur Reinigung von Caprolactam

Gegenstand der Erfindung ist ein Verfahren zur Reinigung von Caprolactam, das durch Beckmannsche Umlagerung erhalten worden ist, bei dem man Rohcaprolactam mit Lösungsmitteln extrahiert, den Extrakt unter Zusatz von Alkali destilliert und reines Caprolactam abtrennt und die alkalischen Rückstände aufarbeitet.

Bei der Destillation von Caprolactam fallen erhebliche Mengen an alkalischen Rückständen an, die entsorgt werden müssen. Da diese Rückstände noch erhebliche Mengen an Caprolactam enthalten, ist es angezeigt, dieses aus dem Rückstand zu gewinnen und zu verwerten. Solches Caprolactam enthält jedoch erhebliche Mengen an Verunreinigungen, die beim Zurückführen in die Destillationsstufe nicht entfernt werden können. Insbesondere wird durch das zurückgeführte Caprolactam die UV-Absorption, die eine Masszahl für Verunreinigungen ist, und die Permanganat Titrationszahl des reinen Caprolactams beeinträchtigt. Aus der Japanischen Patentveröffentlichung 11 332/66 ist bekannt, dass man den alkalischen Destillationsrückstand mit rauchender Schwefelsäure behandelt. Aufgrund der Salzbildung mit den in den Rückständen enthaltenen Alkalien kommt es zu Verkrustungen beim Destillieren, die zu Störungen Anlass geben. Ferner bleibt offen, an welcher Stelle das Caprolactam dem Reinlactam zugegeben werden kann, ohne dass Qualitätseinbussen auftreten.

Es war deshalb die technische Aufgabe gestellt, die Reinigung von Caprolactam so durchzuführen, dass Caprolactam, das aus Rückständen zurückgewonnenen wird ohne die Gefahr einer Anreicherung von Verunreinigungen wieder in die Aufarbeitung zurückgeführt werden kann.

Diese Aufgabe wird gelöst in einem Verfahren zur Reinigung von Caprolactam, das durch Beckmannsche Umlagerung erhalten worden ist, bei dem man Rohcaprolactam mit Lösungsmitteln extrahiert, den Extrakt unter Zusatz von Alkali destilliert und reines Caprolactam abtrennt und aus dem alkalischen Destillationsrückstand weiteres Caprolactam durch Destillation gewinnt, wobei man aus dem alkalischen Destillationsrückstand in einer ersten Stufe bei einer Sumpftemperatur von 130 bis 160°C unter einem Druck von 1,33 bis 13,3 mbar Caprolactam abdestilliert und dieses in die Destillationsstufe zurückführt, den so erhaltenen Rückstand in einer zweiten Stufe bei einer Sumpftemperatur von 140 bis 180°C unter einem Druck von 1,33 bis 13,3 mbar destilliert und das Destillat in einer dritten Stufe mit Schwefelsäure, Phosphorsäure oder stark sauren Ionenaustauschern behandelt und in die Extraktionsstufe zurückführt.

Das neue Verfahren hat den Vorteil, dass die grösstmögliche Menge an Caprolactam aus den alkalischen Destillationsrückständen gewonnen wird. Ferner gelingt es nach dem neuen Verfahren eine Anreicherung von schwer zu beseitigenden Verunreinigungen in den Reinigungsstufen zu vermeiden.

Das zu reinigende Caprolactam wird durch Beckmannsche Umlagerung mit Schwefeltrioxid enthaltender Schwefelsäure erhalten. Ein geeignetes Verfahren wird beispielsweise beschrieben in der US-PS 39 14 217. Nach Neutralisation des Umlagerungsgemisches erhält man Roh-Caprolactam als ölige Phase die abgetrennt und gereinigt wird.

Das Roh-Caprolactam wird zunächst mit Lösungsmitteln extrahiert. Geeignete Lösungsmittel sind beispielsweise Aromaten, wie Benzol oder Toluol. Besonders bewährt hat sich Benzol als Lösungsmittel. Vorteilhaft verfährt man hierbei so, dass man Rohlactam mit dem Lösungsmittel im Gegenstrom behandelt, wobei man im oberen Teil einer Extraktionskolonnne Rohlactam und im unteren Teil Benzol zuführt. Oben an der Kolonne erhält man eine benzolische Lösung von Caprolactam und am unteren Ende eine Verunreinigungen enthaltende wässrige Lösung. Es hat sich bewährt, wenn man im oberen Teil der Extraktionszone zusätzlich Wasser zuführt und einen Teil der am unteren Ende der Extraktionszone anfallenden Verunreinigungen enthaltenden wässrigen Lösung in die Extraktionszone zurückführt um eine möglichst konzentrierte wässrige Lösung zu erhalten. Je Gew.-Teil Rohlactam verwendet man in der Regel die 2- bis 12fache Gewichtsmenge insbesondere 2,5- bis 10fache Gewichtsmenge an Lösungsmittel. Die Extraktion wird beispielsweise bei Temperaturen von 40 bis 65°C durchgeführt. Hierbei hält man in der Regel Atmosphärendruck oder einen schwach erhöhten Druck, z.B. bis 1,5 bar ein. Ein geeignetes Verfahren wird beispielsweise beschrieben in der DE-AS 26 56 182 oder DE-PS 1 194 863.

Die so erhaltene Lösung von Caprolactam, die in der Regel 20 bis 26 Gew.-% Caprolactam enthält sowie Wasser entsprechend der Löslichkeit von Wasser in dem verwendeten Lösungsmittel, wird unter Zusatz von Alkali destilliert. Wegen der leichten technischen Zugänglichkeit verwendet man zweckmässig Natriumhydroxid, das in der Regel als 5 bis 25 gew.-%ige Lösung zugesetzt wird. In der Regel setzt man 0,05 bis 0,5 Gew.-% (berechnet als NaOH), bezogen auf zu reinigendes Caprolactam zu. Die Caprolactamlösung wird nun durch fraktionierte Destillation aufgetrennt und Reinlactam abgezogen. Vorteilhaft führt man die Destillation 3stufig durch, wobei man zunächst in der ersten Kolonne über Kopf Wasser abtrennt, in einer zweiten und dritten Kolonne über Kopf die Leichtsieder entnimmt, Reincaprolactam in der dritten Kolonne am Seitenabzug entnimmt und aus dem Sumpf der dritten Kolonne alkalische Destillationsrückstände abzieht. Die Destillation wird in der Regel bei Sumpftemperaturen von 120 bis 150°C und unter Drücken von 1,33 bis 13,3 mbar (1 bis 10 Torr) durchgeführt.

Erfindungsgemäss destilliert man nun aus dem alkalischen Destillationsrückstand in einer ersten Stufe bei einer Sumpftemperatur von 130 bis 160°C bei einem Druck von 1,33 bis 13,3 mbar (1-10 Torr) Caprolactam ab. Das so erhaltene Caprolactam wird wieder der Destillationsstufe des Reinlactams zugeführt, d.h. in den Zulauf zu den Destillationskolonnen. Der verbleibende Destillationsrückstand, der in der Regel immer noch zwischen 80 und 98 Gew.-% Caprolactam enthält, wird nunmehr in einer Destillierblase zweckmässig unter Rühren auf Temperaturen von 140 bis 180°C bei Drücken von 1,33 bis 13,3 mbar (1-10 Torr) erhitzt und destilliert. Der verbleibende alkalische Rückstand, der weniger als 8 Gew.-% Caprolactam enthält wird entsorgt. Das anfallende Destillat wird nun in einer dritten Stufe mit Schwefelsäure, Phosphorsäure oder stark sauren Ionenaustauschern behandelt.

Vorteilhaft führt man nach einer möglichen Arbeitsweise die Behandlung so durch, dass man dem geschmolzenen Caprolactam 0,1 bis 1 Gew.-% konzentrierte Schwefelsäure oder Phosphorsäure zufügt und dann Caprolactam bei einer Temperatur von 120 bis 180°C im Vakuum bei 1,33-13,3 mbar (1 bis 10 Torr) abdestilliert. Nach einer anderen bewährten Arbeitsweise leitet man die Caprolactamschmelze über einen starksauren Ionenaustauscher. Als stark saure Ionenaustauscher eignen sich beispielsweise sulfonierte Polystyrole. Vorteilhaft hält man hierbei eine Temperatur von 70 bis 90°C ein. Die Verweilzeit der Caprolactamschmelze im Ionenaustauscher beträgt in der Regel 10 Minuten bis 90 Minuten.

Ein wesentliches Merkmal der Erfindung ist es, dass man das Caprolactam nach der Behandlung mit Schwefelsäure, Phosphorsäure oder stark sauren Ionenaustauschern nicht der Destillation sondern in die Extraktionsstufe, d.h. in die Zufuhrleitung zur Extraktionsstufe zurückführt.

Diese Arbeitsweise ermöglicht es, nunmehr auch die Beckmannsche Umlagerung bei einer niedrigeren Temperatur, vorteilhaft 80 bis 130°C zu betreiben, obwohl bei niedrigeren Temperaturen bei der Beckmannschen Umlagerung mehr Verunreinigungen entstehen, während andererseits die Ausbeute von Caprolactam bei der Beckmannschen Umlagerung bei tieferer Temperatur steigt. Insgesamt gelingt es somit, die schwer zu beseitigenden Verunreinigungen problemlos auszuschleusen, das Caprolactam aus den Rückständen wieder den Reinigungsstufen zuzuführen und darüber hinaus die Beckmannsche Umlagerung so zu betreiben, dass insgesamt eine höhere Ausbeute an Caprolactam erzielt wird.

Caprolactam wird zur Herstellung von Polycaprolactam verwendet.

Das Verfahren nach der Erfindung sei an folgenden Beispielen veranschaulicht.

Beispiel 1

In einem Mischkreis werden 5386 kg/h Cyclohexanonoxim, das 4,2 Gew.-% Wasser enthält, in Gegenwart von 5625 kg/h Oleum, das 32 Gew.-% freies Schwefeltrioxid enthält, bei einer mittleren Reaktionstemperatur von 118°C umgelagert. Das entstandene an Schwefelsäure gebundene Rohcaprolactam wird durch Neutralisation mit 2092 kg/h gasförmigem Ammoniak unter Mitverwendung einer nahezu gesättigten wässrigen Ammonsulfatlösung in Freiheit gesetzt und von der Ammoniumsulfatlösung abgetrennt. Die 70 gew.-%ige wässerige Rohcaprolactamlösung extrahiert man in einer Siebbodenkolonne mit 16 340 kg/h Benzol bei 58°C und erhält nach Abdestillieren des Lösungsmittels 5300 kg/h Extrakt-Caprolactam als 95 gew.-%ige wässrige Lösung. Nach Zugabe von 12 kg/h 25 gew.-%iger Natronlauge wird zunächst bei einem Druck von 53,2 mbar (40 Torr) das Wasser abdestilliert. Anschliessend werden bei 4 mbar (3 Torr) unter Abzug eines Vorlaufes von 200 kg/h am Kolonnenkopf und Austrag eines Sumpfes von 600 kg/h am Boden der Kolonne 5000 kg/h Reincaprolactam im Seitenabzug abgetrennt.

Aus dem ausgetragenen Sumpf werden in einer Füllkörperkolonne bei einer Sumpftemperatur von 142°C und einem Druck von 4 mbar (3 Torr) 400 kg/h abdestilliert und in die Hauptdestillationsstufe zurückgeführt. Am Boden der Sumpf-Destillationskolonne trägt man 200 kg/h Rückstand aus und führt ihn einem Dünnschichtverdampfer zu. Bei einer Sumpftemperatur von 168°C werden bei 6,65 mbar (5 Torr) 175 kg/h Kopfprodukt abgezogen und in einer Vorlage gesammelt. Der Sumpfabzug des Dünnschichtverdampfers enthält 1,5 kg/h Caprolactam, 20,5 kg/h Schwersieder und 3 kg/h Alkali und wird verworfen.

Das Kopfprodukt läuft unter Zugabe von 0,35 kg/h 96 gew.-%iger Schwefelsäure in einen Rührkessel, wo es bei einer Sumpftemperatur von 136°C im Vakuum (4-6,65 mbar; 3 bis 5 Torr) destilliert wird. Nach 180 Stunden unterbricht man den Kesselzulauf und destilliert aus. Das Destillat hat eine UV-Kennzahl von 25 bis 30, während die des Zulaufes bei 150 liegt. Das so an UV-aktiven Verunreinigungen abgereicherte Destillat (174 kg/h) wird in die Extraktionsstufe zurückgeführt. Der Rückstand (240,5 kg) enthält 60,5 kg $H_2SO_4$ und 180 kg Caprolactam sowie Verunreinigungen. Es wird nach Neutralisation mit Natronlauge verworfen. Das auf diese Weise erhaltene Reinlactam hat eine UV-Kennzahl von 3,5 bis 4,0. Wird jedoch auf die Zwischenschaltung dieser sauren Destillation verzichtet, erhält man Reinlactam mit einer UV-Kennzahl von 5 bis 6.

Beispiel 2

Man arbeitet wie in Beispiel 1 beschrieben, destilliert jedoch das Destillat der 2. Stufe nicht über Schwefelsäure ab, sondern lässt das Destillat bei einer Temperatur von 85°C über einen stark sauren Ionenaustauscher vom Typ ®Amberlyst 15 (vernetztes sulfoniertes Polystyrol) laufen. Während das Ausgangsmaterial eine UV-Kennzahl von 150 zeigte, wies das über dem Ionenaustauscher behandelte Lactam eine UV-Zahl von 70 auf.

Die UV-Zahl wird wie folgt ermittelt:

Prinzip: Im Spektralbereich von 360 bis 270 nm wird die Absorption des Caprolactams gemessen und nach Umsetzung in einer Kennzahl ausgedrückt.

Analysengeräte: 1 registrierendes Einstrahlspektralphotometer (Carl Zeiss DMR/10) 1 Steilbrustgefäss 80 ml, 2 Quarz-Küvetten mit Deckel 1,0 cm lang (Schichtdicke 1,0 cm).

Vorschrift: 30 g Caprolactam werden in einem Steilbrustgefäss in 30 g bidestilliertem Wasser aufgelöst, und auf Zimmertemperatur abgekühlt. Mit dieser Lösung wird eine Küvette bis zur Eichmarke gefüllt. Die zweite Küvette wird mit dem gleichen bidestillierten Wasser gefüllt und stellt die Vergleichslösung dar.

Nun werden beide Küvetten mit den Deckeln verschlossen, die geschliffenen Flächen mit Seidenpapier gereinigt und in die Küvettenhalter eingesetzt. Dann wird gemäss Geräteanleitung das Spektrum zwischen 370 nm und 260 nm aufgenommen. Die Registriergeschwindigkeit beträgt 120 nm/Minute. Die Extinktionsmessung wird im Messbereich 1 ausgeführt.

Ist die Aufnahme beendet, wird von 270 bis 360 nm alle 10 nm eine Markierung auf dem Papier angebracht.

Auswertung: Aus dem Diagramm werden die Extinktionen bei 270, 280, 290, 300, 310, 320, 330, 340, 350 und 360 nm abgelesen und addiert.

Die Summe der 10 Extinktionswerte wird mit 20 multipliziert und ergibt die UV-Kennzahl. Die UV-Kennzahl wird also immer auf 100%iges Caprolactam und auf eine Schichtdicke von 10 cm bezogen.

## Patentansprüche

1. Verfahren zur Reinigung von Caprolactam, das durch Beckmannsche Umlagerung erhalten worden ist, bei dem man Rohcaprolactam mit Lösungsmitteln extrahiert, den Extrakt unter Zusatz von Alkali destilliert und reines Caprolactam abtrennt und aus dem alkalischen Destillationsrückstand weiteres Caprolactam durch Destillation gewinnt, dadurch gekennzeichnet, dass man aus dem alkalischen Destillationsrückstand in einer ersten Stufe bei einer Sumpftemperatur von 130 bis 160°C unter einem Druck von 1,33 bis 13,3 mbar Caprolactam abdestilliert und dieses in die Destillationsstufe zurückführt, den so erhaltenen Rückstand in einer zweiten Stufe bei einer Sumpftemperatur von 140 bis 180°C unter einem Druck von 1,33 bis 13,3 mbar destilliert und das Destillat in einer dritten Stufe mit Schwefelsäure, Phosphorsäure oder stark sauren Ionenaustauschern behandelt und dann in die Extraktionsstufe zurückführt.

2. Verfahren nach Anspruch 1, dadurch gekennzeichnet, dass die saure Behandlung in der dritten Stufe mit 0,1 bis 1 Gew.-% Schwefelsäure oder Phosphorsäure (bezogen auf Caprolactam) erfolgt und Caprolactam abdestilliert wird.

3. Verfahren nach den Ansprüchen 1 und 2, dadurch gekennzeichnet, dass man von Caprolactam ausgeht, das durch Beckmannsche Umlagerung bei einer Temperatur von 80 bis 130°C erhalten worden ist.

## Claims

1. A process for purifying caprolactam, which das been obtained by Beckmann rearrangement, in which process crude caprolactam is extracted with solvents, the extract is distilled in the presence of alkali and pure caprolactam is isolated and further caprolactam is recovered from the alkaline distillation residue by distillation, characterized in that caprolactam is distilled off from the alkaline distillation residue in a first stage at a bottom temperature of 130-160°C under a pressure of from 1.33 to 13.3 millibars and recycled to the distillation stage, the residue thus obtained is distilled in a second stage at a bottom temperature of 140-180°C under a pressure of from 1.33 to 13.3 millibars, and the distillate is treated with sulfuric acid, phosphoric acid and strongly acid ion exchangers in a third stage, and the treated distillate is then recycled to the extraction stage.

2. A process as claimed in claim 1, characterized in that the acid treatment in the third stage is effected with from 0.1 to 1% by weight (based on caprolactam) of sulfuric acid or phosphoric acid, and caprolactam is distilled off.

3. A process as claimed in claims 1 and 2, characterized in that the starting material is caprolactam which has been obtained by Beckmann rearrangement at from 80 to 130°C.

## Revendications

1. Procédé de purification du caprolactame qui a été obtenu par transposition de Beckmann, selon lequel on extrait du caprolactame brut avec des solvants, on distille l'extrait en ajoutant un alcali et on sépare du caprolactame pur et, du résidu de distillation alcalin, on récupère encore du caprolactame par distillation, caractérisé par le fait que du résidu de distillation alcalin on sépare par distillation, dans un premier stade, à une température de la nappe inférieure de 130 à 160°C, sous une pression de 1,33 à 13,3 mbars, du caprolactame qu'on recycle au stade de distillation, on distille le résidu ainsi obtenu dans un deuxième stade, à une température de la nappe inférieure de 140 à 180°C sous une pression de 1,33 à 13,3 mbars et on traite le distillat, dans un troisième stade, avec de l'acide sulfurique, de l'acide phosphonique ou des échangeurs d'ions fortement acides, puis on le recycle au stade d'extraction.

2. Procédé selon la revendication 1, caractérisé par le fait que le traitement acide, au troisième stade, est effectué avec 0,1 à 1% en poids d'acide sulfurique ou phosphonique (rapporté au caprolactame) et on fait distiller le caprolactame.

3. Procédé selon les revendications 1 et 2, caractérisé par le fait qu'on part de caprolactame qui a été obtenu par transposition de Beckmann à une température de 80 à 130°C.